# EUROPÄISCHE PATENTANMELDUNG

(11) **EP 2 363 090 A2**
(43) Veröffentlichungstag der Anmeldung: **07.09.2011**
(21) Anmeldenummer: 11001764.7
(22) Anmeldetag: 03.03.2011
(51) Int. Cl.: A61B 19/00, A61F 9/007

(54) **Medizinisches Handgerät**

(30) Priorität: 03.03.2010 DE 102010010164
(71) Anmelder: Geuder AG, 69126 Heidelberg (DE)
(72) Erfinder: Geuder, Volker, 69126 Heidelberg (DE); Frauenfeld, Dieter, 69121 Heidelberg (DE); Wolschendorf, Marc, 69121 Heidelberg (DE)
(74) Vertreter: Ullrich & Naumann

(57) **Zusammenfassung**

Ein medizinisches Handgerät, Handstück oder Handgriff zum Anschluss an eine Steuer-/Versorgungseinheit (3) zum Betreiben des Handgeräts (1), insbesondere chirurgisches Handgerät zur Durchführung ophthalmologischer Operationen, wobei das Handgerät (1) Kopplungsmittel zum Anschluss an eine sich zwischen der Steuer-/Versorgungseinheit (3) und dem Handgerät (1) erstreckenden Versorgungs-/Entsorgungsleitung (2) aufweist, ist dadurch gekennzeichnet, dass dem Handgerät (1) oder der Verpackung des Handgeräts (1) Identifizierungsmittel (4) mit einem Identifizierungsmerkmal zugeordnet sind, die durch die Steuer-/Versorgungseinheit (3) lesbar bzw. erkennbar sind und dass nach dem Lesen bzw. Erkennen des Identifizierungsmerkmals eine automatische Einstellung in der Steuer-/Versorgungseinheit in Bezug auf die konkrete Ansteuerung, Versorgung und ggf. Entsorgung des jeweiligen Handgeräts erfolgt.

## Beschreibung

Die vorliegende Erfindung betrifft ein medizinisches Handgerät, Handstück oder Handgriff zum Anschluss an eine Steuer-Nersorgungseinheit zum Betreiben des Handgeräts, insbesondere chirurgisches Handgerät zur Durchführung ophthalmologischer Operationen, wobei das Handgerät Kopplungsmittel zum Anschluss an eine sich zwischen der Steuer-Nersorgungseinheit und dem Handgerät erstreckenden Versorgungs-/Entsorgungsleitung aufweist.

Medizinische Handgeräte der gattungsbildenden Art sind in den unterschiedlichsten Ausführungen aus der Praxis bekannt. So kann es sich dabei beispielsweise um ein chirurgisches Instrument zur Operation am menschlichen Auge handeln, beispielsweise um ein chirurgisches Instrument, wie es aus der EP 1 844 705 B1 bekannt ist. Dort geht es im Konkreten um ein zur Beleuchtung des Inneren des Auges dienendes Instrument, welches von einer Steuer/Versorgungseinheit her über einen Lichtleiter als Versorgungsleitung mit Licht versorgt wird. Solches Instrument lässt sich beispielsweise zur Intraokularbeleuchtung verwenden.

Des Weiteren kann es sich bei dem hier in Rede stehenden Handgerät um ein augenchirurgisches Instrument zum Zertrümmern von Linsen und zum Absaugen von Linsentrümmern handeln, wie es aus der DE 40 08 594 C2 bekannt ist. In diesem Falle wird das Handgerät von der Steuer-/Versorgungseinheit her mit pulsierender Druckluft und Spülflüssigkeit versorgt, wobei die zertrümmerten Linsenteile per Unterdruck abgesaugt werden. Hier dient die Schlauchverbindung zwischen dem Handgerät und der Steuer-/Versorgungseinheit sowohl zur Versorgung als auch zur Entsorgung.

An dieser Stelle sei angemerkt, das es für die erfindungsgemäße Lehre keine Rolle spielt, um welche konkreten medizinischen Handgeräte, Handstücke oder Handgriffe es sich handelt. Vielmehr ist wesentlich, dass das Handgerät über eine Steuer-Nersorgungseinheit gesteuert und versorgt sowie entsorgt wird, wobei als Wirkverbindung eine Versorgungs-/Entsorgungsleitung zwischen der Steuer-Nersorgungseinheit und dem Handgerät vorgesehen ist. Dies kann auch einen elektrischen Stromanschluss umfassen.

Wird an die Steuer-/Versorgungseinheit ein konkretes Handgerät angeschlossen, ist es zunächst erforderlich, die Steuer-/Versorgungseinheit - zum Betrieb des jeweiligen Handgeräts - einzustellen. Dies ist zeitaufwendig und erfordert eine nicht unerhebliche Erfahrung im Umgang mit der Steuer-Nersorgungseinheit. Vor allem aber ist die Einstellung zeitraubend.

Außerdem kommt es in der Praxis häufig vor, dass beispielsweise ein auf dem Gebiet der Ophthalmologie tätiger Operateur eine Steuer-Nersorgungseinheit eines konkreten Herstellers nutzt, jedoch Geräte, Handstücke oder Handgriffe anderer Hersteller anschließt, die sich zwar in einem gewissen Umfange von der Steuer-Nersorgungseinheit steuern, regeln und wie auch immer beaufschlagen, versorgen und entsorgen lassen, jedoch mit hohen Risiken im Betrieb, nämlich in Ermangelung einer 100%igen Adaption. Sofern es während des Betriebs zu einem Versagen des Handgeräts kommt, sind Unfälle in Verlaufe des operativen Eingriffs nicht auszuschließen.

In der Praxis gibt es bereits eine mehr oder weniger praktikable Lösung in Bezug auf die gewollte oder ungewollte Verwendung beliebiger Handgeräte mit einer konkreten Steuer-Nersorgungseinheit, nämlich dahingehend, dass die Handgeräte, Handstücke oder Handgriffe mit einer Art mechanischer Codierung versehen sind, so dass an die geräteseitig gelieferte Versorgungs-/Entsorgungsleitung nur solche Handgeräte ankoppelbar sind, die die passende mechanische Codierung zum Andocken bzw. Ankoppeln aufweisen. Durch Nachahmung entsprechender Anschlussstücke kann eine solche Codierung umgangen werden. Die bekannte Problematik bleibt erhalten.

Der vorliegenden Erfindung liegt daher die Aufgabe zugrunde, ein medizinisches Handgerät, ein Handstück oder einen Handgriff zum Anschluss an eine Steuer-Nersorgungseinheit zum Betreiben des Handgeräts, insbesondere ein chirurgisches Handgerät zur Durchführung ophthalmologischer Operationen, anzugeben, welches gleich mit dem Anschluss an die Steuer-Nersorgungseinheit eine komfortable Handhabung gestattet, und zwar unter Vermeidung umfänglicher Einstellarbeiten am Standgerät. Außerdem soll gewährleistet sein, dass das Handgerät ausschließlich an eine geeignete bzw. dazu passende Steuer-Nersorgungseinheit angeschlossen wird. Mit anderen Worten ist sicherzustellen, dass eine vorgegebene Steuer-Nersorgungseinheit die nur dafür konzipierten Handgeräte "akzeptiert" und nur diese vollumfänglich - mit dem Komfort einer automatischen Einstellung - betreibbar sind.

Die voranstehende Aufgabe ist durch die Merkmale des Patentanspruchs 1 gelöst. Danach ist das erfindungsgemäße medizinische Handgerät dadurch gekennzeichnet, dass dem Handgerät oder der Verpackung des Handgeräts Identifizierungsmittel mit einem Identifizierungsmerkmal zugeordnet sind, die durch die Steuer-/Versorgungseinheit lesbar bzw. erkennbar sind und dass nach dem Lesen bzw. Erkennen des Identifizierungsmerkmals eine automatische Einstellung in der Steuer-/Versorgungseinheit in Bezug auf die konkrete Ansteuerung, Versorgung und ggf. Entsorgung des jeweiligen Handgeräts erfolgt.

Erfindungsgemäß ist erkannt worden, dass es möglich ist, dem Handgerät bzw. dem Handstück oder dem Handgriff oder der Verpackung des Handgeräts, etc. ldentifizierungsmittel zuzuordnen, die ein möglichst eindeutiges Identifizierungsmerkmal beinhalten oder generieren. Das Identifizierungsmerkmal ist durch die Steuer-/Versorgungseinheit lesbar bzw. durch diese erkennbar, wobei erst nach dem Lesen bzw. Erkennen des Identifizierungsmerkmals ein ordnungsgemäßer Betrieb des Handgeräts möglich ist, wenn nämlich das Identifizierungsmerkmal den Vorgaben in der Steuer-/Versorgungseinheit entspricht.

Weiter ist erkannt worden, dass das von der Steuer-/Versorgungseinheit erkannte Identifizierungsmerkmal genutzt werden kann zur automatischen Einstellung in der Steuer-/Versorgungseinheit, und zwar in Bezug auf die konkrete Ansteuerung, Versorgung und ggf. Entsorgung des jeweiligen Handgeräts. Dies bedeutet, dass die Steuer-/Versorgungseinheit aufgrund des Identifizierungsmerkmals den Typ des Handgeräts erkennt, die Ansteuerung mit allen Leistungsmerkmalen gestattet und obendrein eine Voreinstellung oder endgültige Einstellung an der Steuer-/Versorgungseinheit vornimmt, die eine umgehende Handhabung des Handgeräts, ohne weitere Einstellungen an der Steuer-/Versorgungseinheit, gestattet. Aufwendige und zeitraubende Einstellarbeiten sind somit unter Zugrundelegung der erfindungsgemäßen Lehre nicht mehr erforderlich.

In ganz besonders vorteilhafter Weise erfolgt die Einstellung der Steuer-/Versorgungseinheit aufgrund eines erkannten Identifizierungsmerkmals automatisch dahingehend, dass die Steuer-/Versorgungseinheit auf die vom konkreten Handgerät abrufbaren bzw. dort aktivierbaren Funktionen und Leistungen eingestellt wird, zumindest aber bis zum Abruf der jeweiligen Leistung dazu vorbereitet wird. Dies kann bedeuten, dass eine Aspirationspumpe innerhalb der Steuer-Nersorgungseinheit anläuft, zumindest aber in einen Standby-Modus geschaltet wird. In einer der Steuer-/Versorgungseinheit vorgesehenen Unterdruckkammer kann der erforderliche Unterdruck aufgebaut werden. Eine erforderliche Stromversorgung kann ebenfalls vorbereitet bzw. aktiviert werden. Eine Laserlichtquelle kann eingeschaltet werden, bis hin zum Abruf der Lichtleistung. Ein dazwischengeschalteter Schatter kann bis zum Abruf der Leistung die Lichtleitfaser schonen, so dass eine frühzeitige Alterung der Lichtleitfaser wirksam vermieden wird.

Die erfindungsgemäße Lehre bietet einen weiteren Vorteil, nämlich dahingehend, dass nach Erkennen des Identifizierungsmerkmals alle Funktionen und Leistungsstufen der Steuer-/Versorgungseinheit nutzbar sind, nämlich durch eine handgerätespezifische Aktivierung/Freischaltung der Steuer-/Versorgungseinheit. Ein Missbrauch, insbesondere durch unsachgerechte Nutzung nicht passender Handgeräte, Handstücke oder Handgriffe in Verbindung mit einer beliebigen Steuer-/Versorgungseinheit ist insoweit ausgeschlossen.

In vorteilhafter Weise sind in der Steuer-/Versorgungseinheit relevanten Identifizierungsmerkmale zum Abgleich abgelegt, die mit einem jeweiligen ldentifizierungsmerkmal des Handgeräts übereinstimmen müssen.

Im Rahmen einer einfachen Ausgestaltung der erfindungsgemäßen Lehre ist es möglich, dass das Identifizierungsmerkmal durch ein vorzugsweise der Steuer-/Versorgungseinheit zugeordnetes Lesegerät insbesondere lichtoptisch erkennbar und in die Steuer-/Versorgungseinheit einlesbar ist. Bei dem Identifizierungsmerkmal kann es sich beispielsweise um einen Barcode oder dgl. handeln, der beim Erwerb des Handgeräts erhältlich oder aufgrund eines Kaufnachweises über das Internet abrufbar und der Steuer-/Versorgungseinheit zuführbar ist. Eine Online-Verbindung der Steuer-/Versorgungseinheit, beispielsweise über das Internet, mit dem Hersteller der Steuer-Nersorgungseinheit und der Handgeräte ist denkbar, so dass bereits mit dem Abverkauf eines konkreten Handgeräts bzw. der Auslieferung des Handgeräts die Steuer-/Versorgungseinheit - für den Kunden quasi automatisch - mit entsprechenden Identifizierungsmerkmalen ausgestattet wird.

In besonders vorteilhafter und dabei raffinierter Weise sind die Identifizierungsmittel, die letztendlich das Identifizierungsmerkmal generieren oder beinhalten, in dem Handgerät, vorzugsweise im Handgriff des Handgeräts, vorgesehen. Im Konkreten können die Identifizierungsmittel in einem endseitigen Handgerät-Stecker vorgesehen sein, an den die Versorgungs-/Entsorgungsleitung, zumindest aber eine Stromleitung, mit einer entsprechenden Buchse angeschlossen wird. Ebenso ist es denkbar, dass die Identifizierungsmittel sowohl im Handgerät bzw. in dem endseitigen Handgerät-Stecker als auch in einer Anschlussbuchse der Versorgungs-/Entsorgungsleitung bzw. einer elektrischen Leitung oder in einer Buchse in der Steuer-/Versorgungseinheit vorgesehen sind, wobei sich die Identifizierungsmittel beider Bauteile zur Definition des Identifizierungsmerkmals ergänzen. Auch durch diese Maßnahme ist eine weitere Stufe an Sicherheit realisiert.

Im Konkreten ist es von Vorteil, dass im Handgriff bzw. im Handgriff-Stecker ein elektronisches Bauteil, vorzugsweise ein Widerstand, angeordnet ist, das nach Ankopplung an die Versorgungs-/Entsorgungsleitung über eine Konstantstromquelle gespeist und über eine elektrische Schaltung, beispielsweise als Element einer Brückenschaltung oder dgl., von der Steuer-/Versorgungseinheit detektierbar bzw. erkennbar ist. So könnte im Konkreten der Wert des jeweiligen Widerstands zu einem Brückenabgleich führen. Wird ein Handgerät ohne elektrischen Widerstand oder mit einem "falschen" Widerstand angeschlossen, findet der Brückenabgleich nicht statt, so dass aufgrund einer entsprechenden Detektion eine Freischaltung des Handgeräts über die Steuer-/Versorgungseinheit verweigert wird. Ebenso könnte einfach der Widerstand ermittelt und verglichen werden.

Die Stromversorgung und ggf. weitere elektronische Bauteile, abseits des Handgeräts, sind in weiter vorteilhafter Weise in die Steuer-/Versorgungseinheit integriert, wobei eine Integration auch in die Versorgungs-/Entsorgungsleitung, beispielsweise in deren Anschlüsse oder in einer separate Stromleitung bzw. dessen Stecker, vorgesehen sein kann, so dass ein Abgleich zwischen dem Handgerät, der Versorgungs-/Entsorgungsleitung und der Steuer-/Versorgungseinheit in Bezug auf den Hersteller möglich ist. Dies ist von weiterem Vorteil.

Des Weiteren kann ein Mikrocontroller zur Signalverarbeitung und Weiterleitung der ausgewerteten Signale, insbesondere zur Generierung eines Freigabesignals in der Steuer-/Versorgungseinheit, vorgesehen sein. Die Integration einer geeigneten Prozessortechnik, beispielsweise als Bestandteil eines in der Steuer-/Versorgungseinheit ohnehin vorgesehen Prozessors, ist denkbar.

Wie bereits zuvor erwähnt, ist es grundsätzlich denkbar, dass die Steuer-/Versorgungseinheit in Ermangelung eines passenden Identifizierungsmerkmals am bzw. im Handgerät oder bei Detektion eines in der Steuer-/Versorgungseinheit nicht hinterlegten Identifizierungsmerkmals das Handgerät nicht unterstützt. Dies kann bedeutet, dass dem Handgerät jedwede Unterstützung bzw. Versorgung/Entsorgung verweigert wird.

Alternativ ist es denkbar, das die Steuer-/Versorgungseinheit in Ermangelung eines passenden Identifizierungsmerkmals am bzw. im Handgerät oder bei Detektion eines in der Steuer-/Versorgungseinheit nicht hinterlegten Identifizierungsmerkmals das Handgerät zwar aktiviert, jedoch nicht alle Funktionen unterstützt. Auch ist es möglich, dass die Funktionen, beispielsweise betreffend Lichtstärke, Luftdruck, Unterdruck, etc. mit nur reduzierter Leistung aktiviert bzw. unterstützt werden. Eine Art Notbetrieb wäre dann möglich, wobei zur Ausnutzung der gesamten Leistungsfähigkeit der Steuer-/Versorgungseinheit unter Nutzung des Handgeräts ein passendes Handgerät erforderlich ist.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht die prinzipielle Anordnung von Steuer-Nersorgungseinheit, Handgerät und verbindender Versorgungs-/Entsorgungsleitung,

- Fig. 2: in einer schematischen Ansicht, im Sinne eines Blockschaltbildes, im Rahmen eines ersten Ausführungsbeispiels, das Zusammenwirken von Handgerät und Identifizierungsmitteln, die im Handgerät und in der Steuer-/Versorgungseinheit untergebracht sind und
- Fig. 3: in einer schematischen Ansicht, im Sinne eines Blockschaltbildes, im Rahmen eines zweiten Ausführungsbeispiels, das Zusammenwirken eines als Intraokularbeleuchtung angedachten Handgeräts mit einer Konstantstromquelle und einem nachgeschalteten Microcontroller.

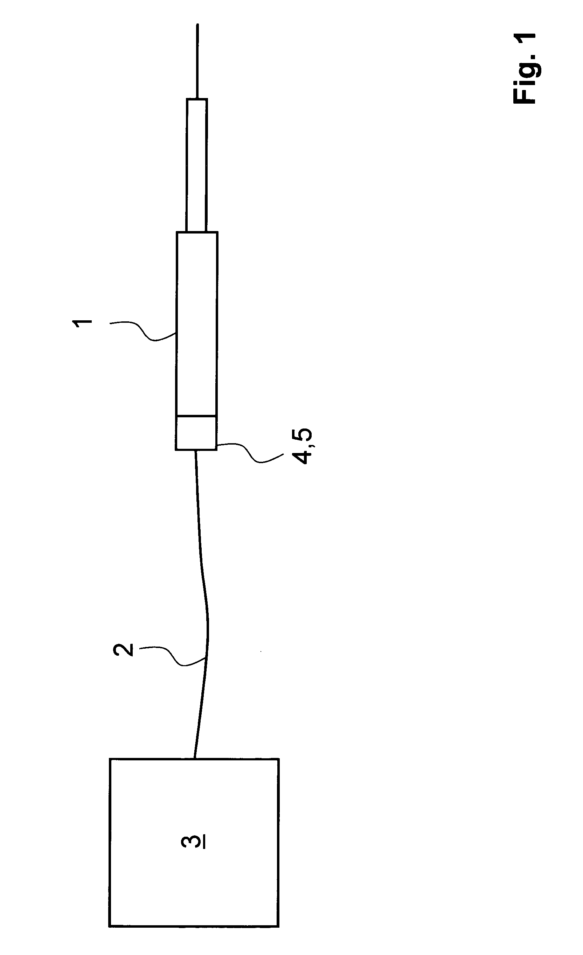

Fig. 1 zeigt in einer vereinfachten schematischen Ansicht Darstellung ein medizinisches Handgerät 1, welches mit einer Steuer-/Versorgungseinheit 3 verbunden ist. Die zuvor genannten Komponenten und Einheiten sind als autarkes System zu verstehen, welches von Außen her lediglich eine Stromversorgung benötigt.

Erfindungsgemäß sind dem Handgerät 1 Identifizierungsmittel 4 zur Ausgabe eines ldentifizierungsmerkmals zugeordnet.

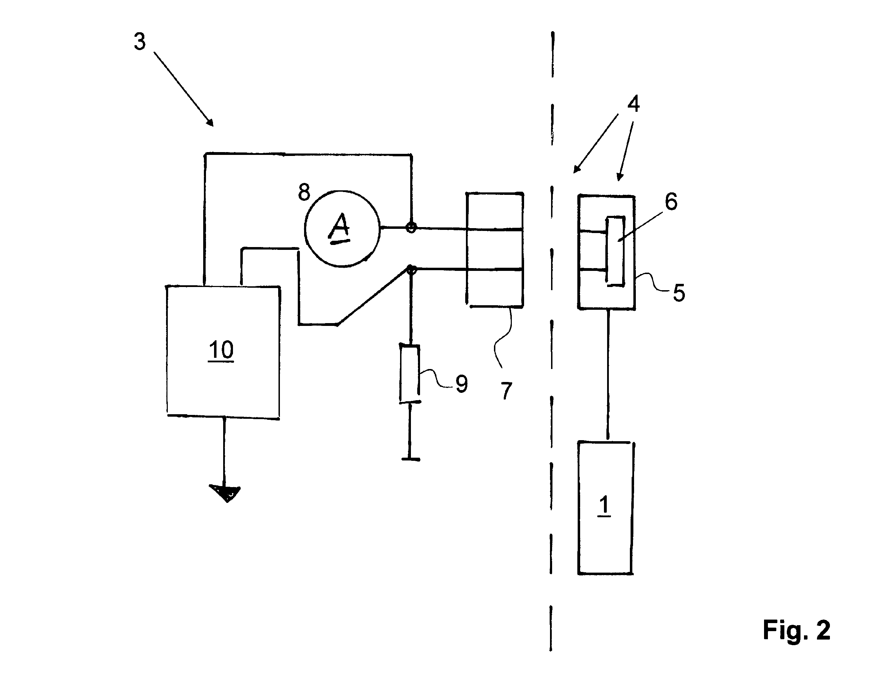

Bei dem in Fig. 2 gezeigten Ausführungsbeispiel umfasst das Handgerät 1 bzw. dessen Handgriff einen Handgriff-Stecker 5, in den ein elektrischer Widerstand 6 integriert ist. Das Handgerät 1 ist durch einen Handgriff-Stecker 5 über die in Fig. 2 nicht gezeigte Versorgungs-/Entsorgungsleitung mit einer Buchse 7 in der Steuer-/Versorgungseinheit 3 verbunden, wobei dazu - abseits der Versorgungs-/Entsorgungsleitung - ein elektrisches Kabel dienen kann, welches sich zwischen dem Handgriff-Stecker 6 und der Buchse 7 erstreckt. Eine Kombination mit der Versorgungs-/Entsorgungsleitung 2 ist möglich.

Von der Steuer-/Versorgungseinheit 3 aus wird die zur Generierung des ldentifizierungsmerkmals dienende elektrische Schaltung mit Konstantstrom versorgt, nämlich über eine Konstantstromquelle 8. Der elektrische Widerstand 6 kann wie auch ein weiterer, zur elektrischen Schaltung gehörender Widerstand 9 Teil einer Brückenschaltung sein, wobei bei entsprechender Ankopplung eines geeigneten elektrischen Widerstands 6 im Handgriff-Stecker 5 ein Brückenabgleich erfolgt. Auch ist es denkbar, dass lediglich der elektrische Widerstand 6 im Handgriff-Stecker 5 - als solcher - detektiert wird. Beliebige Auslegungen der elektrischen/ elektronischen Schaltung sind denkbar.

Zur Signalaufbereitung und Verarbeitung dient ein Mikrocontroller 10, so dass von dort aus eine Aktivierung der Steuer-/Versorgungseinheit 3 erfolgt.

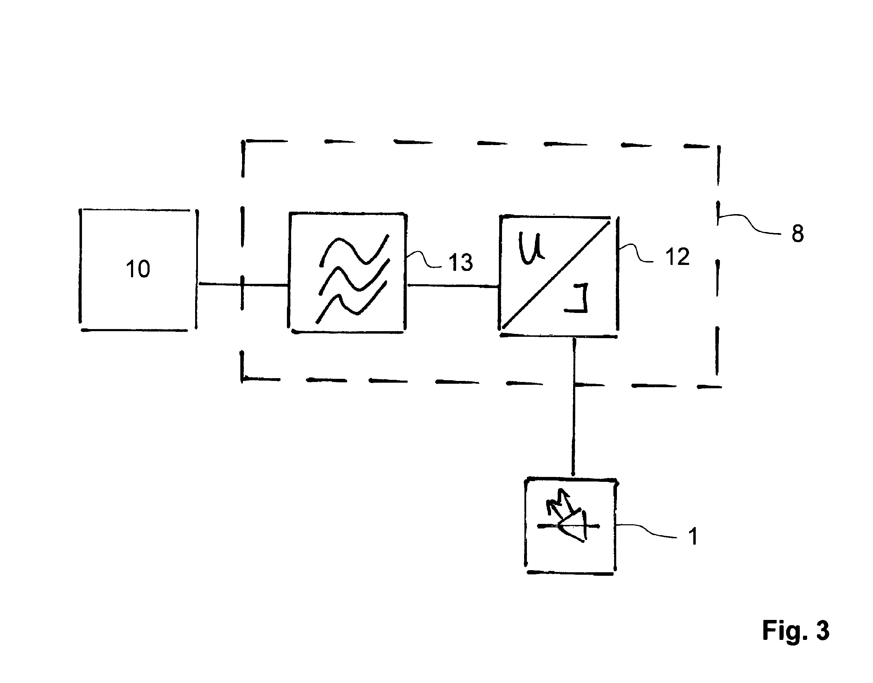

Fig. 3 zeigt ein weiteres Ausführungsbeispiel eines erfindungsgemäßen Handgeräts am Beispiel einer LED-Handlampe 11 zur Intraokularbeleuchtung, die über eine Konstantstromquelle 8 mit einem Spannungs-/Stromwandler 12 und einem Filter 13 gespeist wird. Ein Mikrocontroller 10 ist nachgeschaltet, der ein detektiertes ldentifizierungsmerkmal entsprechend der Beschreibung zu Fig. 2 zur Einstellung der nachgeschalteten Steuer-/Versorgungseinheit 3 nutzt, wobei dies über den der Steuer-Versorgungseinheit 3 zugeordneten Prozessor erfolgen kann.

Auch hier ist wesentlich, dass die Erkennung des Identifizierungsmerkmals genutzt wird, nämlich in erster Linie zur Voreinstellung oder Einstellung der Steuer-Nersorgungseinheit auf das konkrete medizinische Handgerät.

Hinsichtlich weiterer Merkmale, die sich den Figuren nicht entnehmen lassen, sei zur Vermeidung von Wiederholungen auf den allgemeinen Teil der Beschreibung verwiesen.

Schließlich sei angemerkt, dass das die voranstehend erörterten Ausführungsbeispiele lediglich der beispielhaften Erörterung der beanspruchten Lehre dienen, diese jedoch nicht auf die Ausführungsbeispiele einschränken.

### Bezugszeichenliste

- 1: medizinisches Handgerät, Handgriff
- 2: Versorgungs-/Entsorgungsleitung
- 3: Steuer-/Versorgungseinheit
- 4: Identifizierungsmittel
- 5: Handgriff-Stecker
- 6: elektrischer Widerstand (im Handgriff-Stecker)
- 7: Buchse
- 8: Gleichstromquelle/Konstantstromquelle
- 9: elektrischer Widerstand (in der Steuer-/Versorgungseinheit)
- 10: Mikrocontroller
- 11: LED-Handlampe
- 12: Spannungs-/Stromwandler
- 13: Filter

## Patentansprüche

1. Medizinisches Handgerät, Handstück oder Handgriff zum Anschluss an eine Steuer-/Versorgungseinheit (3) zum Betreiben des Handgeräts (1), insbesondere chirurgisches Handgerät zur Durchführung ophthalmologischer Operationen, wobei das Handgerät (1) Kopplungsmittel zum Anschluss an eine sich zwischen der Steuer-/Versorgungseinheit (3) und dem Handgerät (1) erstreckenden Versorgungs-/Entsorgungsleitung (2) aufweist,
**dadurch gekennzeichnet, dass** dem Handgerät (1) oder der Verpackung des Handgeräts (1) Identifizierungsmittel (4) mit einem Identifizierungsmerkmal zugeordnet sind, die durch die Steuer-/Versorgungseinheit (3) lesbar bzw. erkennbar sind und dass nach dem Lesen bzw. Erkennen des Identifizierungsmerkmals eine automatische Einstellung in der Steuer-/Versorgungseinheit in Bezug auf die konkrete Ansteuerung, Versorgung und ggf. Entsorgung des jeweiligen Handgeräts erfolgt.

2. Handgerät nach Anspruch 1, **dadurch gekennzeichnet, dass** aufgrund eines erkannten Identifizierungsmerkmals die Steuer-/Versorgungseinheit (2) automatisch auf die vom konkreten Handgerät (1) abrufbaren bzw. aktivierbaren Funktionen und Leistungen eingestellt, zumindest aber vorbereitet wird.

3. Handgerät nach Anspruch 1 oder 2, **dadurch gekennzeichnet, dass** nach dem Erkennen des Identifizierungsmerkmals ein ordnungsgemäßer Betrieb des Handgeräts (1), insbesondere unter Nutzung aller Funktionen und Leistungsstufen, durch eine handgerätspezifische Aktivierung/Freischaltung der Steuer-/Versorgungseinheit (3) möglich ist.

4. Handgerät nach einem der Ansprüche 1 bis 3, **dadurch gekennzeichnet, dass** in der Steuer-/Versorgungseinheit (3) relevante Identifizierungsmerkmale zum Abgleich abgelegt sind.

5. Handgerät nach einem der Ansprüche 1 bis 4, **dadurch gekennzeichnet, dass** das Identifizierungsmerkmal durch ein vorzugsweise der Steuer-/Versorgungseinheit (3) zugeordnetes Lesegerät vorzugsweise lichtoptisch, erkennbar und in die Steuer-/Versorgungseinheit (3) einlesbar ist.

6. Handgerät nach einem der Ansprüche 1 bis 5, **dadurch gekennzeichnet, dass** als Identifizierungsmerkmal eine Barcode oder dgl. dient, der beim Erwerb des Handgeräts (1) erhältlich oder aufgrund eines Kaufnachweises über das Internet abrufbar und der Steuer-/Versorgungseinheit (3) zuführbar ist.

7. Handgerät nach einem der Ansprüche 1 bis 6, **dadurch gekennzeichnet, dass** die Identifizierungsmittel (4) in dem Handgerät (1), vorzugsweise im Handgriff, vorgesehen sind.

8. Handgerät nach Anspruch 7, **dadurch gekennzeichnet, dass** die Identifizierungsmittel (4) in einem endseitigen Handgerät-Stecker (5) vorgesehen sind.

9. Handgerät nach Anspruch 7 oder 8, **dadurch gekennzeichnet, dass** die Identifizierungsmittel (4) sowohl im Handgerät (1) bzw. in dem endseitigen Handgerät-Stecker (5) als auch in einer Anschlussbuchse der Versorgungs-/Entsorgungsleitung (2) vorgesehen sind, wobei sich die Identifizierungsmittel (4) beider Bauteile zur Definition des Identifizierungsmerkmals ergänzen.

10. Handgerät nach einem der Ansprüche 7 bis 9, **dadurch gekennzeichnet, dass** im Handgriff (1) bzw. im Handgriff-Stecker (5) ein elektronisches Bauteil (6), vorzugsweise ein Widerstand, angeordnet ist, das nach Ankopplung an die Versorgungs-/Entsorgungsleitung (2) über eine Gleichspannungsquelle gespeist und über eine elektrische Schaltung, beispielsweise als Element einer Brückenschaltung oder dgl., von der Steuer-/Versorgungseinheit detektierbar bzw. erkennbar ist.

11. Handgerät nach Anspruch 10, **dadurch gekennzeichnet, dass** die Stromversorgung und ggf. weitere elektronische Bauteile (9) in die Steuer-/Versorgungseinheit integriert sind.

12. Handgerät nach Anspruch 10 oder 11, **dadurch gekennzeichnet, dass** ein Mikrocontroller (10) zur Signalverarbeitung und Weiterleitung der ausgewerteten Signale dient.

13. Handgerät nach einem der Ansprüche 1 bis 12, **dadurch gekennzeichnet, dass** die Steuer-/Versorgungseinheit (3) in Ermangelung eines passenden Identifizierungsmerkmals am bzw. im Handgerät (1) oder bei Detektion eines in der Steuer-Nersorgungseinheit (3) nicht hinterlegten Identifizierungsmerkmals das Handgerät (1) nicht unterstützt.

14. Handgerät nach einem der Ansprüche 1 bis 12, **dadurch gekennzeichnet, dass** die Steuer-/Versorgungseinheit (3) in Ermangelung eines passenden ldentifizierungsmerkmals am bzw. im Handgerät (1) oder bei Detektion eines in der Steuer-/Versorgungseinheit (3) nicht hinterlegten Identifizierungsmerkmals nicht alle Funktionen oder Funktionen mit nur reduzierter Leistung aktiviert bzw. unterstützt.
